# EUROPÄISCHE PATENTSCHRIFT

(11) **EP 0 832 924 B1**
(45) Veröffentlichungstag und Bekanntmachung des Hinweises auf die Patenterteilung: **13.10.2004**
(21) Anmeldenummer: 97114425.8
(22) Anmeldetag: 21.08.1997
(51) Int. Cl.: C08L 23/02

(54) **Polyolefinformmasse zur Herstellung von Vliesen**
Polyolefin composition for the preparation of non-wovens
Composition de polyoléfine pour fabriquer des non-tissés

(30) Priorität: 29.08.1996 DE 19634894
(43) Veröffentlichungstag der Anmeldung: 01.04.1998
(73) Patentinhaber: Basell Polyolefine GmbH, 50389 Wesseling (DE)
(72) Erfinder: Winter, Andreas, Dr., 61479 Glashütten (DE); Vollmar, Annette, Dl., 60385 Frankfurt (DE); Fraaije, Volker, Dr., 60325 Frankfurt (DE); Brekner, Michael-Joachim, Dr., 65760 Eschborn (DE); Siemon, Manfred, Dr., 36251 Bad Hersfeld (DE)

(56) Entgegenhaltungen:
- EP-A- 0 527 589
- EP-A- 0 588 208
- US-A- 5 516 848
- PATENT ABSTRACTS OF JAPAN vol. 095, no. 009, 31.Oktober 1995 & JP 07 145202 A (MITSUI TOATSU CHEM INC), 6.Juni 1995,

## Beschreibung

Die Erfindung bezieht sich auf eine Polyolefinformmasse zur Herstellung hochfester Vliese und auf ein wirtschaftliches und umweltfreundliches Verfahren zur Herstellung von Vliesen.

Generell ist die Verwendung von Polyolefinen für die Herstellung von Fasern, Filamenten und Vliesen, die oft auch als nonwoven fabrics bezeichnet werden, bekannt. Eine solche Verwendung wird in EP-A-0 028 844 beschrieben. So sind auch Schmelzspinnverfahren bekannt, bei denen Polyolefine, insbesondere Polypropylene, die nach der Polymerisation mittels eines peroxidischen Abbauschrittes in einem zusätzlichen Verfahrensschritt für diese Anwendungen konditioniert werden müssen, eingesetzt werden. Diese als CR-Polymere (CR = controlled rheology) bezeichneten Formmassen weisen gravierende Nachteile auf: Der zusätzliche Verfahrensschritt verteuert die Produktion der Fasern, Filamente oder Vliese, und durch diesen peroxidischen Abbauschritt entstehen niedermolekulare Fragmente, die einen unangenehmen Geruch aufweisen und im Verarbeitungsprozeß zum Auftreten von sogenanntem Spinnrauch führen.

Ferner führen diese niedermolekularen Anteile auch zu Gelbverfärbungen der Produkte. Weiterhin werden diese Polymeren mit Ti-Katalysatoren hergestellt. Prinzipbedingt enthalten sie größere Anteile an ataktischem Polyolefin, beispielsweise im Falle von Polypropylenrohstoffen ataktisches Polypropylen, sowie Cl- und Ti-Bestandteile in größerer Menge als Katalysatorrückstände. Während Halogen in der Restasche zu Korrosion der Verarbeitungsmaschinen führt, neigt Ti zur Komplexbildung mit den normalerweise dem Polymer zugesetzten Stabilisatoren. Die Farbe dieser Komplexe ist mitverantwortlich für einen oft beobachtbaren Gelbstich solcher Polymere.

Neben Anteilen an ataktischem Polyolefin enthalten solchermaßen dem Stand der Technik entsprechende Polymere auch noch sogenannte Öle, niedermolekulare isotaktische, gestört isotaktische oder auch völlig ataktische Polymere beziehungsweise Oligomere.

Diese Bestandteile führen bei der Verarbeitung durch Anreicherung beispielsweise in einem Spinnprozeß an der Düsenplatte zu Tropfenbildung und somit zu massiver Störung des Produktionsprozesses durch Verklebung von Fasern oder durch Störungen auf der Faser- bzw. Filamentablage oder durch das Auftreten von starken Fadenabriß-Störungen.

Die Herstellung der Polyolefinrohstoffe mit Metallocenkatalysatoren beseitigen diese Probleme. Es sind Polyolefine mit enger Molmassenverteilung im geforderten Molmassenbereich ohne die Notwendigkeit eines peroxidischen Abbaus direkt in der Polymerisation kostengünstig herstellbar. Die Polymere enthalten keine oligomeren Ölbestandteile und keinen oder nur einen geringen Anteil an ataktischem Polymer. Ein solches Polymer wird für die Faserherstellungsprozesse in EP-A-0 600 461 oder in WO 94/28219 beschrieben.

Über die bessere Verarbeitbarkeit hinaus bieten solche neuartigen Polymere, insbesondere wichtig sind Polypropylene, weitere Vorteile: Die Festigkeit der damit hergestellten Fasern und Filamente ist deutlich höher als bei der Verwendung der herkömmlichen Polyolefine, und der pro Zeiteinheit erzielbare Materialdurchsatz liegt aufgrund höherer möglicher Spinngeschwindigkeit deutlich höher. Darüber hinaus sind Fasern mit geringerer Fadenstärke zugänglich. Die mit Metallocenkatalysatoren hergestellten Polymere zeichnen sich somit durch deutliche ökologische und ökonomische Vorteile aus.

Versuche haben jedoch gezeigt, daß die höheren Festigkeiten der Fasern und Filamente bei der Weiterverarbeitung zu Vliesen im normalerweise angewendeten Thermobondierverfahren nicht zu einem auch deutlich festeren Vlies führen.

Die Herstellung der Einzelkomponenten der Formmassen mittels Metallocenkatalysatoren ist aus EP-A-537 686, EP-A-549 900, EP-A-545 303 und EP-A-576 970 bekannt. Es handelt sich dabei um hochmolekulare isotaktische Polyolefine, von denen insbesondere Polypropylene und propylenhaltige Copolymere zu nennen sind. Die Herstellung von Formmassen ist aus EP-A-588 208 bekannt

Polyolefinformmassen die mindestens zwei unterschiedliche Polyolefine enthalten, sind aus US-A 5 516 848, EP-A-588 208, EP-A-527 589 und JP 07 145202 A bekannt.

Aufgabe der vorliegenden Erfindung besteht darin, eine Polyolefinformmasse mit der Vliesen mit verbesserter hoher Festigkeit hergestellt werden können und ein wirtschaftliches und umweltfreundliches Verfahren zur Herstellung von Vliesen bereitzustellen.

Die Aufgabe wird erfindungsgemäß durch ein Verfahren zur Herstellung von Vliesen (nonwoven fabrics) aus mindestens einer Polyolefinformmasse gelöst, wobei die Polyolefinformmasse mindestens zwei unterschiedliche Polyolefine enthält, die durch Polymerisation oder Copolymerisation von Olefinen der Formel R⁹CH = CHR⁶ hergestellt werden, worin R⁹ und R⁶ gleich oder verschieden sind und ein Wasserstoffatom oder einen Alkylrest mit 1 bis 14 C-Atomen bedeuten oder worin R⁹ und R⁶ mit den sie verbindenden Atomen ein Ringsystem bilden, und 0 bis 60 Gew.-% Ethylen oder eines zweiten Olefins mit obengenannter Bedeutung als Comonomer enthalten. Polypropylenformmassen sind besonders bevorzugt.
bevorzugt 10 bis 40 Gew.-% mindestens eines weiteren Polyolefins mit niedrigerer Isotaktizität. Polyolefin bedeutet dabei bevorzugt Polypropylen.

Die Polyolefinformmassen für die erfindungsgemäße Verwendung zur Herstellung von Vliesen enthalten mindestens ein Polyolefin mit hoher Isotaktizität und 5 bis 60 Gew.-%, bevorzugt 7 bis 50 Gew.-%, besonders bevorzugt 10 bis 40 Gew.-% mindestens eines weiteren Polyolefins mit niedrigerer Isotaktizität. Polyolefin bedeutet dabei bevorzugt Polypropylen.

Anstelle eines Polyolefins mit niedrigerer Isolaktizität kann auch mindestens ein Copolymer als Formmassenbestandteil verwendet werden. Bevorzugt sind solche Copolymere statistisch aufgebaut und bedeuten bevorzugt Propylen/Ethylen- oder Propylen/Hexen-Copolymere mit 0,5 bis 60, bevorzugt 1 bis 50, besonders bevorzugt 1,5 bis 40 Gew.-% Ethylen- bzw. Hexen-Gehalt.

Bei Polyolefinformmassen für das erfindungsgemäße Verfahren, zur Herstellung von Vliesen betragen der MFI (230/2,16) von 5 bis 1000 dg/min, bevorzugt 7 bis 300 dg/min, besonders bevorzugt 10 bis 100 dg/min, die mittels GPC ermittelte Molmasse M_{w} 75 000 bis 350 000 g/mol, bevorzugt 100 000 bis 225 000 g/mol, besonders bevorzugt 120 000 bis 200 000 g/mol, die Polydispersität (M_{w}/Mₙ) von 1,8 bis 3,0, besonders bevorzugt von 2,0 bis 3,0 und die Viskositätszahl von 70 bis 250 cm³/g, bevorzugt 90 bis 200 cm³/g besonders bevorzugt 110 bis 180 cm³/g. Der etherextrahierbare Anteil ist kleiner als 2 Gew.-%, bevorzugt kleiner als 1 Gew.-%, besonders bevorzugt kleiner als 0,5 Gew.-%. Die einzelnen Polymeren unterscheiden sich in der Formmasse in ihrem Schmelzpunkt, ihrer isotaktischen Blocklänge und/oder in ihrem Comonomergehalt.

Die Formmassen für die erfindungsgemäße Verwendung sind so aufgebaut, daß sich die Schmelzpunkte der Einzelkomponenten um mindestens 5°C, bevorzugt 8°C, besonders bevorzugt um mehr als 10°C unterscheiden, eine der Komponenten eine isotaktische Blocklänge von mindestens 40, bevorzugt 50 bis über 200 aufweist und die andere(n) Komponente(n) eine isotaktische Blocklänge von 10 bis 80, bevorzugt 15 bis 70 aufweist, wobei der Unterschied der Blocklängen der Komponenten in der Formmasse mindestens 5, bevorzugt 10, bevorzugt größer 15, sein muß. Alternativ zu der Mischung von Polymeren mit unterschiedlichen Blocklängen kann auch mindestens ein Polymer isotaktische Blocklängen im Bereich von 50 bis über 200 aufweisen und das/die andere(n) Polymer(e) ist (sind) ein Copolymer (Copolymere) mit 0,5 bis 60 Gew.-%, bevorzugt 1 bis 50 Gew.-%, besonders bevorzugt 1,5 bis 40 Gew.-% Comonomergehalt. Bevorzugte Comonomere sind Ethylen und Hexen. Gemeinsames Merkmal der erfindungsgemäß zu verwendenden Polymere ist ein breiter Schmelzbereich.

Eine weitere Ausgestaltung der Erfindung sind Polyolefinformmassen, enthaltend mindestens zwei unterschiedlichen Polyolefine, von denen mindestens ein Polyolefin isotaktisch mit einer isotaktischen Blocklänge von 40 bis 200 aufgebaut ist und das andere Polyolefin syndiotaktisch aufgebaut ist, wobei sich die Schmelzpunkte des syndiotaktisch aufgebauten und des isotaktisch aufgebauten Polyolefins um mindestens 10°C, bevorzugt 15°C unterscheiden.

Eine bevorzugte Ausgestaltung der Erfindung ist eine Formmasse, die Zusatzstoffe enthält. Solche sind insbesondere Nukleierungsmittel wie etwa Talkum, Natriumbenzoat, Stearate oder Sorbitolderivate, Stabilisatoren, Antioxidantien, UV-Absorber, Lichtschutzmittel, Metalldesaktivatoren, Radikalfänger, Gleitmittel, Emulgatoren, Pigmente, optische Aufheller, Flammschutzmittel oder Antistatika.

Die Formmassen können entweder durch Mischen der Einzelkomponenten oder durch direkte Polymerisation hergestellt werden. Das Mischen der Polymeren kann nach einer in der Kunststoffverarbeitung üblichen Methode erfolgen. Eine der Möglichkeiten ist das Sintern in einem schnellaufenden Mischer, eine weitere Möglichkeit ist der Einsatz eines Extruders, bevorzugt mit Misch- und Knetorganen auf der Schnecke, oder es wird ein Kneter, wie er in der Gummi- oder Kautschukindustrie verwendet wird, eingesetzt. Die einfachste Methode besteht im innigen Mischen der Polymerpulver, eventuell zusammen mit oben angeführten Zusätzen, und anschließender Extrusion mit einem in der Kunststoffindustrie gängigen Extruder.

Die direkte Polymerisation wird mit Katalysatormischungen realisiert oder durch mehrstufige Polymerisationen, wobei Temperatur, Druck, Wasserstoffgehalt, Comonomergehalt, Metallocenkatalysatortypen bzw. Zusammensetzungen variiert werden können. Entsprechend dem Stand der Technik wird bei Temperaturen von 0 bis 100°C und bei Monomerdrücken von 1 bis 100 bar in Lösung, in Suspension oder in der Gasphase polymerisiert. Die Metallocenkatalysatoren können als Lösung oder geträgert und auch geträgert und vorpolymerisiert in der Polymerisation eingesetzt werden.

Die Formmassen werden mit Metallocenkatalysatoren hergestellt. Insbesondere werden Zirkonocene, die substituierte Indenylsysteme als Liganden aufweisen, verwendet. Syndiotaktische Komponenten werden mit Fluorenyl/Cp-Zirkonocenen hergestellt.

Die Polymerisation findet somit in Gegenwart eines Katalysators statt, welcher als Übergangsmetallkomponente mindestens zwei Metallocene der allgemeinen Formel (I) enthält 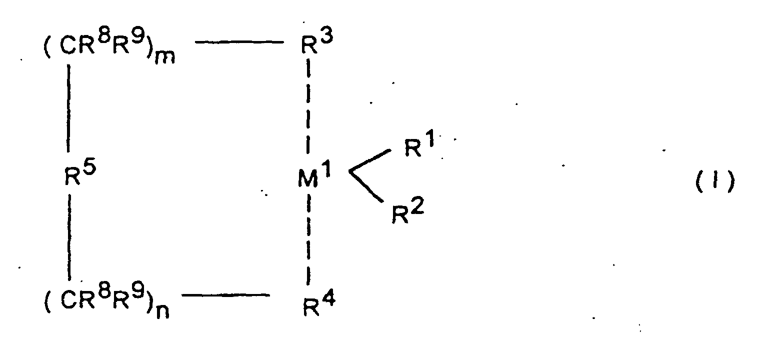 worin
- M¹: Zr, Hf oder Ti bedeutet,
- R¹ und R²: gleich oder verschieden sind und ein Wasserstoffatom, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
- R³ und R⁴: gleich oder verschieden sind, und einen ein- oder mehrkernigen, unsubstituierten oder substituierten Kohlenwasserstoffrest, welcher mit dem Metallatom M¹ eine Sandwichstruktur bilden kann, bedeuten,
- R⁵: 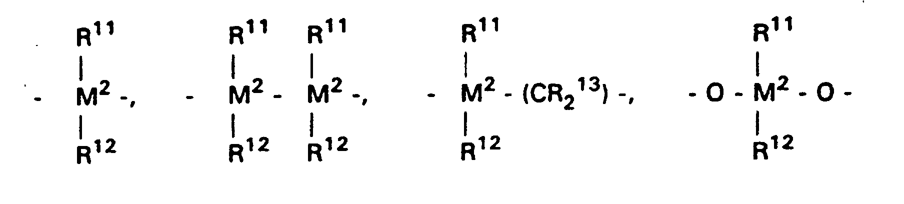 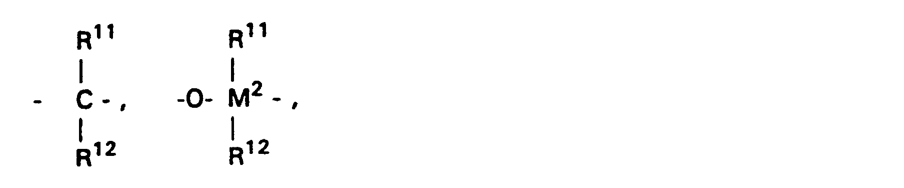 =BR¹¹, =AIR¹¹, -Ge-, -Sn-, -O-, -S-, =SO, =SO₂, =NR¹¹, =CO, =PR¹¹ oder =P(O)R¹¹ ist,
wobei
R¹¹, R¹² und R¹³ gleich oder verschieden sind und ein Wasserstoffatom, ein Halogenatom, eine Si(alkyl)₃-Gruppe, eine Si(aryl)₃-Gruppe, eine N(alkyl)₂-Gruppe, eine N(aryl)₂-Gruppe, eine B(alkyl)₂-Gruppe, eine B(aryl)₂-Gruppe, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden und
M² Silizium, Germanium oder Zinn ist,
- R⁸ und R⁹: gleich oder verschieden sind und die für R¹¹ genannte Bedeutung haben und
- m und n: gleich oder verschieden sind und null, 1 oder 2 sind, wobei m plus n null, 1 oder 2 ist.

Bevorzugt sind Metallocene der allgemeinen Formel (I), worin
- M¹: Zr, Hf oder Ti, bevorzugt Zr und Hf, besonders bevorzugt Zr ist,
- R¹ und R²: gleich oder verschieden sind und ein Wasserstoffatom, eine C₁-C₁₀-, vorzugsweise C₁-C₃-Alkylgruppe, eine C₁-C₁₀-, vorzugsweise C₁-C₃-Alkoxygruppe, eind C₆-C₁₀-, vorzugsweise C₆-C₈-Arylgruppe, eine C₆-C₁₀-, vorzugsweise C₆-C₈-Aryloxygruppe, eine C₂-C₁₀-, vorzugsweise C₂-C₄-Alkenylgruppe, eine C₇-C₄₀-, vorzugsweise C₇-C₁₀-Arylalkylgruppe, eine C₇-C₄₀-, vorzugsweise C₇-C₁₂-Alkylarylgruppe, eine C₈-C₄₀-, vorzugsweise C₈-C₁₂-Arylalkenylgruppe oder ein Halogenatom, vorzugsweise Chlor bedeuten.
- R³ und R⁴: gleiche oder verschiedene, ein- oder mehrkernige, unsubstituierte oder substituierte Kohlenwasserstoffreste bedeuten, die mit dem Metallatom M¹ eine Sandwichstruktur bilden können,
- R⁵: 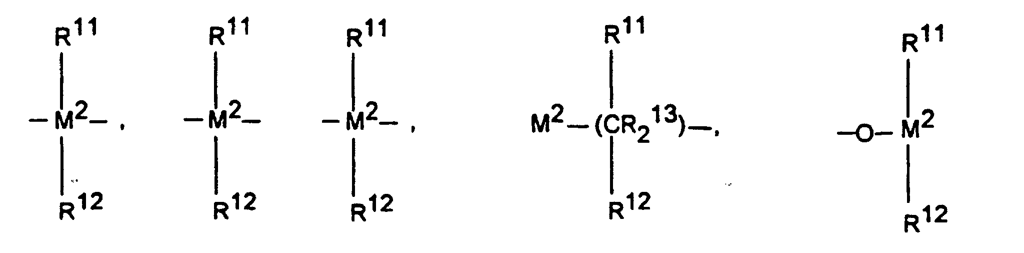 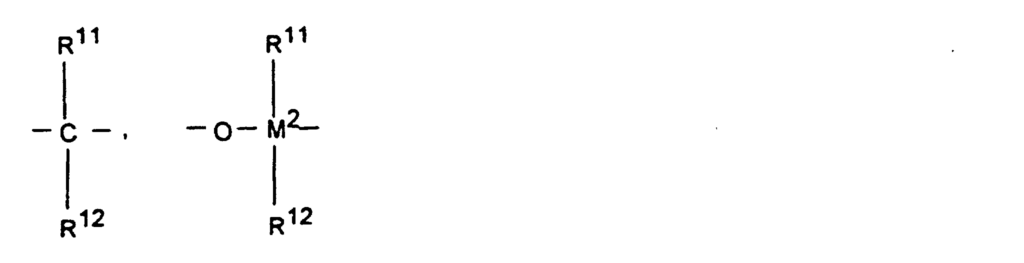 =BR¹¹, =AIR¹¹, -Ge-, -Sn-, -O-, -S-, =SO, =SO₂, =NR¹¹, =CO, =PR¹¹ oder =P(O)R¹¹ bedeutet, wobei
R¹¹, R¹² und R¹³ gleich oder verschieden sind und ein Wasserstoffatom, ein Halogenatom, eine Si(methyl)₃-Gruppe, eine Si(phenyl)₂-Gruppe, eine N(methyl)₂)-Gruppe, eine N(phenyl)₂-Gruppe, eine B(methyl)₂-Gruppe, eine B(phenyl)₂-Gruppe, eine C₁-C₁₀-, vorzugsweise C₁-C₄-Alkylgruppe, insbesondere Methylgruppe, eine C₁-C₁₀-Fluoralkylgruppe, vorzugsweise CF₃-Gruppe, eine C₆-C₁₀-, vorzugsweise C₆-C₈-Arylgruppe, eine C₆-C₁₀-Fluorarylgruppe, vorzugsweise Pentafluorphenylgruppe, eine C₁-C₁₀-, vorzugsweise C₁-C₄-Alkoxygruppe, insbesondere Methoxygruppe, eine C₂-C₁₀-, vorzugsweise C₂-C₄-Alkenylgruppe, eine C₇-C₄₀-, vorzugsweise C₇-C₁₀-Arylalkylgruppe, eine C₈-C₄₀-, vorzugsweise C₈-C₁₂-Arylalkenylgruppe oder eine C₇-C₄₀-, vorzugsweise C₇-C₁₂-Alkylarylgruppe bedeuten, oder R¹¹ und R¹² oder R¹¹ und R¹³ bilden jeweils zusammen mit den sie verbindenden Atomen einen Ring bedeuten und
M² Silizium, Germanium oder Zinn, bevorzugt Silizium und Germanium ist.
- R⁵: ist bevorzugt =CR¹¹R¹², =SiR¹¹R¹², =GeR¹¹R¹², -O-, -S-, =SO, =PR¹¹ oder =P(O)R¹¹,
- R⁸ und R⁹: sind gleich oder verschieden und haben die für R¹¹ genannte Bedeutung und
- m und n: sind gleich oder verschieden und null, 1 oder 2, bevorzugt null oder 1, wobei m plus n null, 1 oder 2, bevorzugt null oder 1 ist.

Die besonders bevorzugten Metallocene der allgemeinen Formel (I) sind solche, bei denen
- M¹: Zirkon ist,
- R¹ und R²: gleich sind und Methyl oder Chlor bedeuten,
- R⁴ und R³: Indenyl, Cyclopentadienyl oder Fluorenyl bedeuten, wobei diese Liganden noch zusätzliche Substituenten in der Bedeutung von R¹¹, R¹² und R¹³ tragen können, wobei die Substituenten unterschiedlich sein können und durch sie verbindene Atome auch Ringe bilden können, im Falle von R⁴ = R³ = Indenyl ist die Substitution besonders bevorzugt,
- R⁵: 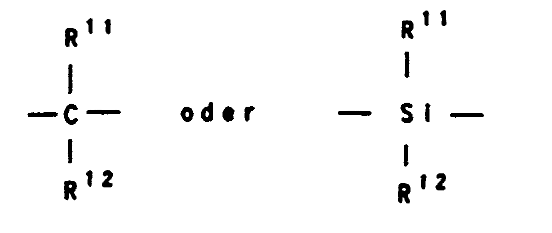 bedeutet und
- n plus m: null oder 1 bedeuten.

Ganz besonders bevorzugt sind die Metallocene der allgemeinen Formel (I), die in den Ausführungsbeispielen aufgeführt sind.

Alkyl steht für geradkettiges oder verzweigtes Alkyl. Halogen (halogeniert) bedeutet Fluor, Chlor, Brom oder Jod, bevorzugt Fluor oder Chlor.

Die chiralen Metallocene werden als Racemat zur Herstellung von hochisotaktischen Polyolefinen eingesetzt. Verwendet werden kann aber auch die reine R- oder S-Form. Mit diesen reinen stereoisomeren Formen ist ein optisch aktives Polymeres herstellbar. Abgetrennt werden sollte jedoch die meso-Form der Metallocene, da das polymerisationsaktive Zentrum (das Metallatom) in diesen Verbindungen wegen Spiegelsymmetrie am Zentralmetall nicht mehr chiral ist und daher kein hochisotaktisches Polymeres erzeugen kann. Wird die meso-Form nicht abgetrennt, entsteht neben isotaktischen Polymeren auch ataktisches Polymer. Für bestimmte Anwendungen - weiche Vliese beispielsweise - kann dies durchaus wünschenswert sein. Die Trennung der Stereoisomeren ist im Prinzip bekannt. Metallocene mit formaler C_{S}-Symmetrie sind zur Herstellung von syndiotaktischen Polyolefinen geeignet, ihr Einsatz wird bevorzugt für die Herstellung weicher, griffsympathischer Vliese empfohlen.

Die Metallocene der allgemeinen Forme (I) können prinzipiell nach folgendem Reaktionsschema hergestellt werden:

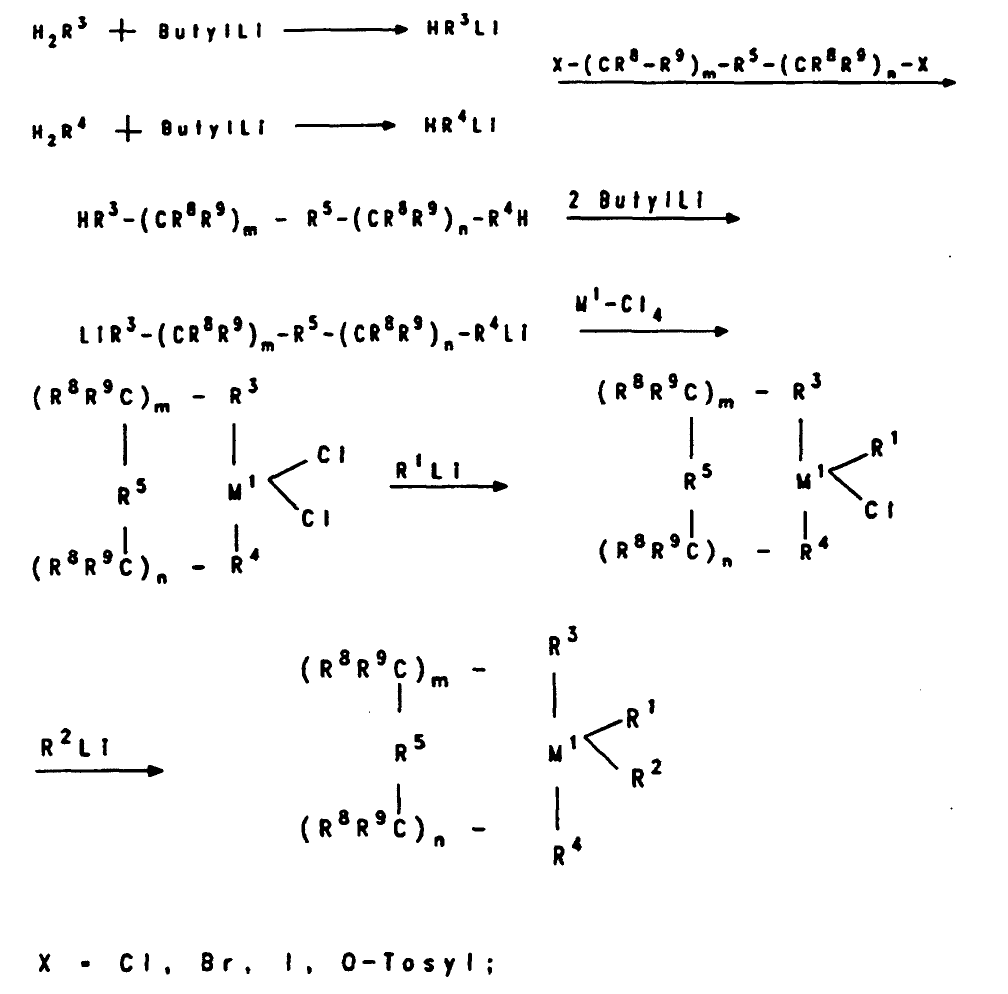

Als Cokatalysator wird ein Aluminoxan der Formel (II) für den linearen Typ und/oder der Formel (III) für den cyclischen Typ 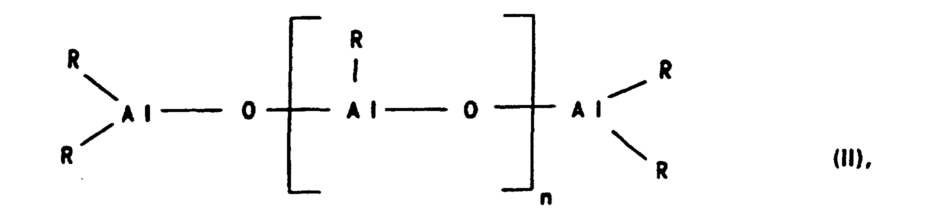 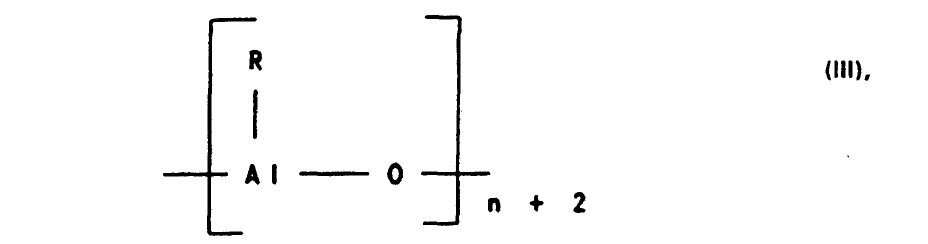 verwendet, wobei in den Formeln (II) und (III) die Reste
- R: gleich oder verschieden sein können und eine C₁-C₆-Alkylgruppe, eine C₁-C₆-Fluoralkylgruppe, eine C₆-C₁₈-Arylgruppe, eine C₆-C₁₈-Fluorarylgruppe oder Wasserstoff bedeuten und n eine ganze Zahl von 0 bis 50, bevorzugt 10 bis 35, bedeutet und die Aluminiumoxan-Komponente zusätzlich eine Verbindung der Formel AIR₃ enthalten kann.

Sind in den Formeln (II) und (III) die Reste
- R: gleich, bedeuten sie bevorzugt Methyl, Isopropyl, Isobutyl, Phenyl oder Benzyl, besonders bevorzugt ist Methyl.

Sind in den Formeln (II) und (III) die Reste
- R: unterschiedlich, bedeuten sie bevorzugt Methyl und Wasserstoff oder alternativ Methyl und Isobutyl, wobei Wasserstoff oder Isobutyl bevorzugt zu 0,01 bis 40 % (Zahl der Reste R) enthalten sind.

Das Aluminoxan kann auf verschiedene Arten nach bekannten Verfahren hergestellt werden. Eine der Methoden ist beispielsweise, daß eine Aluminiumkohlenwasserstoffverbindung und/oder eine Hydridoaluminiumkohlenwasserstoffverbindung mit Wasser (gasförmig, fest, flüssig oder gebunden - beispielsweise als Kristallwasser) in einem inerten Lösungsmittel (wie z.B. Toluol) umgesetzt wird. Zur Herstellung eines Aluminoxans mit verschiedenen Alkylgruppen R werden entsprechend der gewünschten Zusammensetzung zwei verschiedene Aluminiumtrialkyle (AlR₃ + AIR'₃) mit Wasser umgesetzt (vgl. S. Pasynkiewicz, Polyhedron 9 (1990) 429 und EP-A-302 424). Die genaue Struktur der Aluminoxane II und III ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Statt des Aluminoxans kann bei der Polymerisation als Cokatalysator eine Mischung bestehend aus Aluminoxan und AlR₃ verwendet werden, wobei R die oben angeführte Bedeutung hat.

Es ist möglich, die Metallocene vor dem Einsatz in der Polymerisationsreaktion jeweils getrennt oder zusammen als Mischung mit einem Aluminoxan der Formel (II) und/oder (III) vorzuaktivieren. Dadurch wird die Polymerisationsaktivität deutlich erhöht und die Kornmorphologie des Polymers verbessert.

Die Voraktivierung der Metallocene wird in Lösung vorgenommen. Bevorzugt werden dabei die Metallocene als Feststoff in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol oder ein C₆-C₁₀-Kohlenwasserstoff verwendet.

Die Konzentration des Aluminoxans in der Lösung liegt im Bereich von etwa 1 Gew.-% bis zur Sättigungsgrenze, vorzugsweise von 5 bis 30 Gew.-%, jeweils bezogen auf die Gesamtlösung. Die Metallocene können in der gleichen Konzentration eingesetzt werden, vorzugsweise werden sie jedoch in einer Menge von 10⁻⁴ - 1 mol pro mol Aluminoxan eingesetzt. Die Voraktivierungszeit beträgt 1 Minute bis 60 Stunden, vorzugsweise 5 bis 60 Minuten. Man arbeitet bei einer Temperatur von -78 °C bis 100 °C, vorzugsweise 0 °C bis 70 °C.

Die Metallocene können auch vorpolymerisiert oder auf einen Träger aufgebracht werden. Zur Vorpolymerisation wird bevorzugt das (oder eines der) in der Polymerisation eingesetzte(n) Olefin(e) verwendet.

Geeignete Träger sind beispielsweise Silikagele, Aluminiumoxide, festes Aluminoxan, Kombinationen von Aluminoxan auf einem Träger wie beispielsweise Silikagel oder andere anorganische Trägermaterialien. Ein geeignetes Trägermaterial ist auch ein Polymerpulver, bevorzugt Polyolefinpulver in feinverteilter Form.

Eine weitere mögliche Ausgestaltung des Verfahrens besteht darin, daß man an Stelle oder neben eines Aluminoxans eine salzartige Verbindung der Formel RₓNH₄₋ₓBR'₄ oder der Formel R₃PHBR'₄ als Cokatalysator verwendet. Dabei sind x = 1,2 oder 3, R = Alkyl oder Aryl, gleich oder verschieden, und R' = Aryl, das auch fluoriert oder teilfluoriert sein kann. In diesem Fall besteht der Katalysator aus dem Reaktionsprodukt der Metallocene mit einer der genannten Verbindungen (vgl. EP-A-277 004).

Zur Entfernung von im Olefin vorhandener Katalysatorgifte ist eine Reinigung mit einem Aluminiumalkyl, beispielsweise Al(i-Butyl)₃, AlMe₃ oder AlEt₃ vorteilhaft. Diese Reinigung kann sowohl im Polymerisationssystem selbst erfolgen, oder das Olefin wird vor der Zugabe in das Polymerisationssystem mit der Al-Verbindung in Kontakt gebracht und anschließend wieder abgetrennt.

Die Polymerisation oder Copolymerisation wird in bekannter Weise in Lösung, in Suspension oder in der Gasphase, kontinuierlich oder diskontinuierlich, ein- oder mehrstufig bei einer Temperatur von vorzugsweise 0 bis 100 °C, durchgeführt. Polymerisiert oder copolymerisiert werden Olefine der Formel R⁹-CH = CH-R⁶. In dieser Formel sind R⁹ und R⁶ gleich oder verschieden und bedeuten ein Wasserstoffatom oder einen Alkylrest mit 1 bis 14 C-Atomen. R⁹ und R⁶ können jedoch auch mit den sie verbindenden C-Atomen einen Ring bilden und 0 bis 60 Gew.-% Ethylen oder ein zweites Olefin mit obengenannter Bedeutung als Comonomer enthalten. Beispiele für Olefine entsprechend der Formel R⁹-CH = CH-R⁶ sind Ethylen, Propylen, 1-Buten, 1-Hexen, 4-Methyl-1-penten, 1-Octen, Norbornen Ethylidennorbornen oder Norbornadien. Insbesondere werden Propylen und Ethylen oder Hexen polymerisiert. Als Molmassenregler wird, falls erforderlich, Wasserstoff zugegeben. Der Gesamtdruck im Polymerisationssystem beträgt 1 bis 100 bar. Bevorzugt ist die Polymerisation in dem technisch besonders interessanten Druckbereich von 5 bis 64 bar.

Dabei werden die Metallocene in einer Konzentration, bezogen auf das Übergangsmetall, von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan oder die Mischung Aluminoxan/AIR₃ wird in einer Konzentration von 10⁻⁵ bis 10⁻¹ mol, vorzugsweise 10⁻⁴ bis 10⁻² mol pro dm³ Lösemittel bzw. pro dm³ Reaktorvolumen verwendet. Prinzipiell sind aber auch andere Konzentrationen möglich.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff, als solcher sei beispielsweise Butan, Pentan, Hexan, Heptan, Decan, Isooctan, Cyclohexan, Methylcyclohexan, genannt.

Weiterhin kann eine Benzin- oder hydrierte Dieselölfraktion benutzt werden. Brauchbar ist auch Toluol. Bevorzugt wird im flüssigen Monomeren polymerisiert. Werden inerte Lösemittel verwendet, werden die Monomeren gasförmig oder flüssig zudosiert.

Die Dauer der Polymerisation ist beliebig, da das erfindungsgemäß zu verwendende Katalysatorsystem einen nur geringen zeitabhängigen Abfall der Polymerisationsaktivität zeigt.

Das Verfahren zeichnet sich dadurch aus, daß mit den beschriebenen Metallocenen im technisch besonders interessanten Temperaturbereich zwischen 40 °C und 80°C mit hoher Polymerisationsaktivität Polymere mit erfindungsgemäßem Eigenschaftsprofil für die Herstellung hochfester Vliese (nonwoven fabrics) hergestellt werden können.

Die Formmassen mit dem beschriebenen Eigenschaftsprofil können in allen Spinnverfahren eingesetzt werden. Bekannt sind Kurzspinnverfahren oder Kompaktspinnverfahren mit Rechteckspinndüsenpakten oder Runddüsen oder Langspinnverfahren mit Anblasung über den Blasschacht.

Bei diesen Verfahren erfolgt Aufschmelzen der Polymerformmasse in einem Extruder, Verspinnen durch Lochdüsen, Anblasen mit Luft zur Abkühlung, der Abzug. Danach erfolgt Verstrecken der so gebildeten Fasern/Faserbündel/Kabel über Galetten und eine eventuelle weitere Nachverstreckung und thermische Nachbehandlung in Heizkanälen. Die Fasern/Faserbündel/Kabel werden nach Trocknung anschließend in Stauchkammern, Krimper oder Stauchkräuselmaschinen gekräuselt. Die Kräuselung wird dann thermisch fixiert. In einer Schneideeinrichtung wird die Kräuselung auf die gewünschte Faser- bzw. Stapelfaserlänge geschnitten. Das Schnittgut wird anschließend zu Ballen verpreßt. Alternativ kann das Schnittgut auch direkt der Weiterverarbeitung zum Faservlies zugeführt werden. Generell kann der gesamte Prozeß beginnend 17 bei der Extrusion bis zur Ballenpressung oder Weiterverarbeitung einstufig oder mehrstufig durchgeführt werden.

Die weitere Verarbeitung zu Faservliesen beinhaltet eine erneute Zerteilung des Preßballens (Ballenöffnung im sogenannten Bale opener) und die Herstellung einer Vorvliesbahn in einer Formierungszone, beispielsweise einer Karde. Dieses noch unverfestigte Faservlies wird dann über ein Förderband einer eventuell vorhandenen Vorheizzone und schließlich einem Kalander zugeführt, wo durch thermische Fixierung (Thermobondierung) das Vlies verfestigt wird. Abschließend wird nach Randbeschnitt im Wickler auf Rollen aufgewickelt oder alternativ weiter konfektioniert und dann aufgewickelt. Vor der Thermobondierungsstufe können dem Vorvlies noch weitere Vliesschichten zugeführt werden, wie das Aufbringen von Meltblownfasern.

Die im Thermobondierverfahrensschritt mit dem erfindungsgemäß anzuwendenden Polymeren notwendigen Fixiertemperaturen liegen im Bereich von 90 °C bis 200°C, bevorzugt 100 °C bis 180°C, besonders bevorzugt 110 °C bis 170°C. Die Wahl dieser Temperatur ist so zu gestalten, daß die Temperatur hoch genug ist, um eine einwandfreie Fixierung zu gewährleisten. Der maximale obere Temperaturpunkt ist abhängig von der Vliesdurchlaufgeschwindigkeit durch die Thermofixierwalzen (Kontaktzeit) und wird durch beginnendes Verkleben des Vlieses mit den temperierten Walzen (Gravurwalzen) angezeigt. Für einen sicheren Anlagenlauf ist die Temperatur dann wieder etwas abzusenken. Die Vliesgeschwindigkeit in der Thermofixiereinrichtung, die die Kontaktzeit für den Faserverschmelz-/ Faserfixiervorgang determiniert, ist in Abhängigkeit von der Vliesdicke variabel gestaltbar und beträgt 8 m/min bis 500 m/min, bevorzugt 15 m/min bis 350 m/min, besonders bevorzugt 25 m/min bis 300 m/min. Generell können Vliese auf Basis der erfindungsgemäßen Polymere mit Vliesgewichten (Flächengewichten) von 5 g/m² bis 200 g/m², bevorzugt 5 g/m² bis 150 g/m², besonders bevorzugt 10 g/m² bis 100 g/m², hergestellt werden.

Um eine einwandfreie Thermofixierung zu gewährleisten, können dickere Vliese (Vliese mit höherem Flächengewicht, insbesondere solche mit Flächengewichten > 50 g/m²) vor der eigentlichen Thermobondierung thermisch vorbehandelt, bzw. vorgeheizt werden. Von Vorteil ist dies insbesondere dann, wenn dickere Vliese mit sehr hohen Verarbeitungsgeschwindigkeiten, entsprechend kurzen Kontaktzeiten in der Thermofixiereinheit, verarbeitet werden sollen.

Die durch die Thermofixierwalzeneinheit auf dem Vlies erzeugte Verschweißpunktfläche soll 10 % bis 40 %, bevorzugt 12 % bis 25 %, bezogen auf die Vliesgesamtfläche betragen. Der Liniendruck der Thermofixierwalzen beträgt entsprechend dem Stand der Technik je nach Auslegung der Anlage 20 dN/cm bis 200 dN/cm und ist durch die Anwendung der erfindungsgemäßen Formmasse nicht eingeschränkt.

Alternativ kann das Vlies auch direkt in einem Einstufenprozeß hergestellt werden. Die Spinnvliesenanlagen arbeiten entweder nach dem Saugluft- oder Unterdruckverfahren oder nach dem Druckluftverfahren. Der eigentliche Spinnprozeß, die Verstreckung und die Abkühlung der Fasern erfolgt in einem Spinnschacht. Die Fasern werden nicht geschnitten, sondern als Endlosfasern auf einem Ablageband der Thermofixiereinheit zugeführt. Die Kühlung der Filamente/Fasern erfolgt durch Blasluft im oberen Teil des Spinnschachtes. Der Abzug der Filamente/Fasern erfolgt durch geführte Luftströmungen beispielsweise nach dem Saugluftverfahren oder über Saug- bzw. Injektordüsen. Die Filamente werden dann auf ein laufendes Ablageband in gleichmäßiger Flächendicke mit Hilfe eines Diffusors verlegt und dem oben bereits beschriebenen Thermofixierkalander zugeführt.

Erfindungsgemäß ist die Verwendung eines Vlieses als Hygienevlies, Agrarvlies, Filtervlies, Bauvlies, Unterspannvlies im Textilbereich und Schutzanzugsvlies vorgesehen.

Generell zeichnen sich die erfindungsgemäßen Polymere gegenüber solchen vom Stand der Technik beschriebenen Polymeren dadurch aus, daß sich mit ihnen auch bei kurzen Thermofixierkontaktzeiten eine verbesserte Vliesfixierung erzielen läßt und somit eine höhere Vliesfestigkeit realisiert werden kann.

Die vorliegende Erfindung wird anhand der nachstehenden Beispiele näher erläutert.

### Beispiele

Im ersten Teil werden zunächst die Herstellung der speziellen Polymertypen beispielhaft beschrieben. Im zweiten Teil wird die erfindungsgemäße Verwendung der Polymere im Spinnprozeß näher erläutert.

Es bedeuten:

| | |
|---|---|
| VZ = | Viskositätszahl in cm³/g |
| M_{w} = | Molmassengewichtsmittel in g/mol ermittelt durch Gelpermeations-Chromatographie |
| M_{w}/Mₙ = | Polydispersität |
| MFR(230/2.16) = | Schmelzindex gemessen nach DIN 53735 bei 230°C Schmelzetemperatur und 2.16 kg Belastungsgewicht, Einheit in dg/min gleichbedeutend mit g/10 min |
| Schmpt. = | DSC (20°C/min) |
| Titer = | Fadengewicht in g/10 000 m (dtex) |
| Vliesfestigkeit = | N/5 cm |
| Dehnung = | in % jeweils gemessen in Maschinenlaufrichtung (längs, MD) und quer dazu (CD) |

Herstellung von Formmassen für die erfindungsgemäßen Verwendungen durch Blenden/Mischen von Polymerkomponenten auf Metallocenkatalysatorbasis hergestellt.

### Beispiel 1

800 kg Polymer 1 und 200 kg Polymer 2 (mit Metallocenkatalysatoren hergestellte Polypropylenpulver) wurden gemischt, mit 2 kg Pentaerythrityltetrakis [3(3,5-di-t-butyl-4-hydroxy-phenyl)propionat] gegen chemischen Abbau unter Extrusionsbedingungen stabilisiert und in einem Doppelschneckenextruder ZSK 53 (Fa.Werner und Pfleiderer) extrudiert und anschließend granuliert. Die Temperaturen in den Heizzonen waren 150°C (Einzug), 210°C, 250°C, 250°C, 250°C, 280°C und 215°C (Düsenplatte), die Massetemperatur im Extruder war 258°C, (gemessen im Schneckenvorraum), die Extruderschnecken rotierten mit 120 Upm. Die für die Mischung verwendeten Basispolymeren hatten folgende Eigenschaften:

### Polymer 1

VZ = 149 cm³/g; MFR (230/2,16) = 28 dg/min; M_{w} = 139 500 g/mol; M_{w}/Mₙ = 2,1; Schmelzpunkt = 150°C

### Polymer 2

VZ = 155 cm³/g; MFR (230/2,16) = 27,5 dg/min; M_{w} = 141 000 g/mol; M_{w}/Mₙ = 2,2; Schmelzpunkt = 139°C

Die durch Extrusion hergestellte Formmasse wies folgende Daten auf: VZ = 153 cm³/g; MFR (230/2,16) = 28 dg/min; M_{w} = 140 500 g/mol; M_{w}/Mₙ = 2,0; breiter Schmelzbereich mit Schulter bzw. Maxima bei 136°C und 152°C. Der etherextrahierbare Anteil im Polymer (ataktischer Anteil) betrug 0,23 Gew.-%.

### Beispiel 2

Es wurde verfahrenwie in Beispiel 1, Polymer 1 wies jedoch folgende Eigenschaften auf: VZ = 160 cm³/g; MFR = 26 dg/min; M_{w} = 149 500 g/mol; M_{w}/Mₙ = 2,4; Schmelzpunkt = 162°C

Die durch Extrusion hergestellte Formmasse wies folgende Daten auf: VZ = 156 cm³/g; MFR (230/2,16) = 26,5 dg/min; M_{w} = 145 000 g/mol; M_{w}/Mₙ = 2,4; breiter Schmelzbereich mit Schulter bzw. Maxima bei 137°C und 160°C. Der etherextrahierbare Anteil im Polymer (ataktischer Anteil) betrug 0,2 Gew.-%.

### Beispiel 3

Es wurde verfahren wie in Beispiel 1, Polymer 2 war jedoch ein statistisches Propylen/Ethylen-Copolymer mit 2,9 Gew.-% Ethylengehalt; VZ = 165 cm³/g; M_{w} = 152 500 g/mol; M_{w}/Mₙ = 2,0; Schmelzpunkt 129°C, MFR (230/2,16) = 26,7 dg/min. Das Mischungsverhältnis Polymer 1 : Polymer 2 war 850 kg : 150 kg.

Die durch Extrusion hergestellte Formmasse wies folgende Daten auf: VZ = 161 cm³/g; MFR (230/2,16) = 27 dg/min; M_{w} = 146 500 g/mol; M_{w}/Mₙ = 2,5; sehr breiter Schmelzbereich mit einer ausgeprägten Schulter bei 125-135°C und einem Maximum bei 151 °C; etherextrahierbarer Anteil im Polyer < 0,33 Gew.-%.

### Beispiel 4

Es wurde verfahren wie in Beispiel 1, die verwendeten Polymeren waren jedoch in folgender Weise aufgebaut:

### Polymer 1

VZ = 194 cm³/g; MFR (230/2,16) = 12 dg/min; M_{w} = 229 000 g/mol; M_{w}/Mₙ = 2,4; Schmelzpunkt = 151°C.

### Polymer 2

VZ = 80 cm³/g; MFR (230/2,16) = 470 dg/min; M_{w} = 89 500 g/mol; M_{w}/Mₙ = 2,0; Schmelzpunkt = 134°C.

Die durch Extrusion einer Mischung von 700 kg Polymer 1 und 300 kg Polymer 2 hergestellte Formmasse wies folgende Daten auf: VZ = 165 cm³/g; MFR (230/2,16) = 25 dg/min; M_{w} = 184 500 g/mol; M_{w}/Mₙ = 3,4; breiter Schmelzbereich mit Schulter bei 130 bis 140°C und einem Maximum bei 147°C. Etherextrahierbarer Anteil war 0,39 Gew.-%.

### Herstellung von Formmassen für die erfindungsgemäßen Anwendungen in Direktpolymerisation

### Beispiel 5

Ein trockener, 150 dm³-Reaktor wurde mit Propylen gespült und bei 20°C mit 70 dm³ eines entaromatisierten Benzinschnitts mit dem Siedebereich 100 - 120°C, 60 dm³ flüssigem Propylen und 150 cm³ toluolischer Methylaluminoxanlösung (entsprechend 250 mmol Al, Molmasse nach hygroskopischer Bestimmung 1120 g/mol) befüllt. Die Temperatur wurde dann auf 50°C einreguliert. Im Gasraum wurde ein Wasserstoffgehalt von 0,05 Vol.-% eingestellt (während der Polymerisation wird der Gehalt durch laufendes Nachdosieren von Wasserstoff konstant gehalten). 8,5 mg rac-Me₂Si(2-methyl-1-indenyl)₂ZrCl₂ und 3,8 mg rac-Me₂Si(2,5,6-trimethyl-1-indenyl)₂ZrCl₂ wurden gemischt und der Feststoff in 25 cm³ toluolischer Methylaluminoxanlösung (42 mmol Al) gelöst und nach 15 Minuten in den Reaktor gegeben. Durch Kühlung wurde das Polymerisationssystem 15 h bei 52°C gehalten. Die Polymerisation wurde durch Zugabe von 2,5 bar CO₂-Gas gestoppt und das gebildete Polymer (27,2 kg) auf einer Drucknutsche vom Suspensionsmedium abgetrennt. Die Trocknung des Produktes erfolgte 24 h bei 80°C/200 mbar. Das Polymerpulver von 5 in gleicher Weise durchgeführter Polymerisationen wurde mit 250 g Pentaerythrityltetrakis [3-(3,5-di-t-butyl-4-hydroxyphenyl)-propionat] gegen chemischen Abbau versetzt, gemischt und in einem Doppelschneckenextruder ZSK 28 (Fa. Werner und Pfleiderer) extrudiert und dann granuliert. Die Temperaturen in den Heizzonen waren 150°C (Einzug), 200°C, 240°C, 250°C (Düsenplatte), die Drehzahl der Extruderschnecken war 150 Upm, die Massetemperatur war 245°C.

Es wurden 130 kg Granulat erhalten, das folgende Eigenschaften aufwies: VZ = 196 cm³/g; MFR (230/2,16) = 9 dg/min; M_{w} = 228 500 g/mol; M_{w}/Mₙ = 2,3; breiter Schmelzbereich mit Maxima bei 138°C und 152°C. Der etherextrahierbare Anteil im Polymer (ataktischer Anteil) betrug 0,1 Gew.-%.

### Beispiel 6

Beispiel 5 wurde mit 6,0 mg des Metallocens rac-Dimethylsilandiylbis(2-mehyl-4-naphthyl-1-indenyl)zirkondichlorid und 4,5 mg des Metallocens rac-Ethylidenbis(2,5,6-trimethyl-1-indenyl)zirkondichlorid wiederholt. Die im System verwendete Wasserstoffmenge war 1,5±0,1 Vol.-% und die Polymerisationszeit 10 h. Es wurden 26,5 kg Polymer erhalten. Wie in Beispiel 5 wurde das Polymer von 5 in gleicher Weise durchgeführten Polymerisationen homogenisiert und zu Granulat verarbeitet, das folgende Eigenschaften aufwies: VZ = 209 cm³/g; MFR (230/2,16) = 10 dg/min; M_{w} = 214 000 g/mol; M_{w}/Mₙ = 2,9; sehr breiter Schmelzbereich ohne erkennbares Hauptmaximum mit einem Schmelzbeginn bei ca. 50°C und einem Ende des Schmelzvorgangs bei ca. 180°C.

### Beispiel 7

### Darstellung des geträgerten Katalysatorsystems

3,44 g (7,2 mmol) rac-Dimethylsilandiylbis(2-methyl-1-indenyl)zirkondichlorid und 3,94 g (7,4 mmol) rac-Dimethylsilandiylbis(2,5,6-trimethyl-1-indenyl) zirkondichlorid wurden bei Raumtemperatur in 736 cm³ (2,66 mol Al) 30 %iger toluolischer Methylaluminoxan-Lösung (Albemarle Corporation, Baton Rouge, Louisiana, USA) gelöst. Der Ansatz wurde mit 1850 cm³ Toluol verdünnt und 10 min bei 25°C gerührt. In diese Lösung wurden 664 g SiO₂ (Silica Typ MS 948, W.R. Grace, Davison Chemical Division, Baltimore, Maryland, USA, Porenvolumen 1,6 ml/g, calciniert bei 800°C) langsam eingetragen. Das Verhältnis Volumen der Lösung zum Gesamtporenvolumen des Trägermaterials betrug 2,5. Nach beendeter Zugabe wurde der Ansatz 5 min bei Raumtemperatur gerührt. Anschließend wurde der Ansatz innerhalb von 5 h bei 40°C unter Vakuum bis zur Trockne eingeengt und der Rückstand 10 h bei 25°C und 10⁻³ mbar getrocknet. Es wurden 906 g eines frei fließenden, orangefarbenen Pulvers erhalten, das laut Elementaranalyse 0,15 Gew.-% Zr und 7,9 Gew.-% Al enthielt.

### Polymerisationstest

Ein trockener 16 dm³-Reaktor, der zunächst mit Stickstoff und anschließend mit Propen gespült worden war, wurde mit 10 dm³ flüssigem Propen gefüllt. Als Scavenger wurden 8 cm³ 20 %iger Triethylaluminium-Lösung in Varsol (Witco) zugesetzt und der Ansatz 15 min bei 30°C gerührt. Anschließend wurde eine Suspension von 1,3 g des geträgerten Metallocen-Katalysators in 20 cm³ Exxsol in den Reaktor gegeben, auf die Polymerisationstemperatur von 65°C aufgeheizt und das Polymerisationssystem 1 h bei 65°C gehalten. Die Polymerisation wurde durch Zusatz von 20 cm³ Isopropanol gestoppt, das überschüssige Monomer abgegast und das erhaltene Polymer im Vakuum getrocknet. Es resultierten 2,6 kg Polypropylen-Pulver. Die Katalysatoraktivität betrug 122 kg PP / (mmol Zrxh) oder 2 kg PP / (g Katxh).

Das dargestellte isotaktische Polypropylen wies die folgenden Eigenschaften auf: VZ = 176 cm³/g; MFR (230/2,16) = 14,5 dg/min; M_{w} = 189 500 g/mol; M_{w}/Mₙ = 2,2; breiter Schmelzbereich mit Maxima bei 133°C und 147°C. Etherextrahierbare Anteile 0,25 Gew.-%, Schüttdichte des Polymerpulves war 390 g/dm³, gute Rieselfähigkeit.

Für die Verarbeitungsversuche wurden in einer kontinuierlich betriebenen Pilotanlage in vergleichbarer Weise 1100 kg des obigen Polymertyps hergestellt.

### Beispiel 8

Die Darstellung des geträgerten Metallocen-Katalysators erfolgte analog Beispiel 7, aber mit 5,24 g (1 mmol) rac-Dimethylsilandiylbis(2-methyl-1-idenyl) zirkondichlorid und 1,92 mg (3,6 mmol) rac-Dimethylsilandiylbis(2,5,6-trimethyl-1-indenyl)zirkondichlorid. Es resultierten 890 g eines frei fließenden orangefarbenen Pulvers, das laut Elementaranalyse 0,16 Gew.-% Zr und 8,0 Gew.-% Al enthielt.

### Polymerisationstest

Die Polymerisation wurde analog zu Beispiel 7 durchgeführt. Es resultierten 2,9 kg Polypropylen-Pulver. Die Katalysatoraktivität betrug 127 kg PP / (mmol Zrxh) oder 2,2 kg PP / (g Katxh).

Das dargestellte isotaktische Polypropylen wies die folgenden Eigenschaften auf: VZ = 186 cm³/g; MFR (230/2,16) = 12,5 dg/min; M_{w} = 199 500 g/mol; M_{w}/Mₙ = 2,3; breiter Schmelzbereich mit Maxima bei 134°C (breite Schulter) und 147°C. Etherextrahierbare Anteile 0,2 Gew.-%, Schüttdichte des Polymerpulves war 380 g/dm³, gute Rieselfähigkeit.

Für die Verarbeitungsversuche wurden in einer kontinuierlich betriebenen Pilotanlage in vergleichbarer Weise 1000 kg des obigen Polymertyps hergestellt.

### Beispiel 9

Die Darstellung des geträgerten Metallocen-Katalysators erfolgte analog Beispiel 7, aber mit 3,02 g (4,8 mmol) rac-Dimethylsilandiylbis(2-methyl-4-phenyl-1-idenyl) zirkondichlorid und 5,22 g (9,8 mmol) rac-Dimethylsilandiylbis(2,4,6-trimethyl-1-indenyl)zirkondichlorid. Es resultierten 902 g eines frei fließenden orangefarbenen Pulvers, das laut Elementaranalyse 0,16 Gew.-% Zr und 8,1 Gew.-% Al enthielt.

### Polymerisationstest

Die Polymeriation wurde analog zu Beispiel 7 durchgeführt, mit dem Unterschied, daß pro dm³ Flüssigpropylen 0,25 Ndm³ Wasserstoff in den Reaktor gegeben wurden. Es resultierten 1,8 kg Polypropylen-Pulver. Die Katalysatoraktivität betrug 79 kg PP / (mmol Zrxh) oder 1,4 kg PP / (g Katxh). Das dargestellte isotaktische Polypropylen wies die folgenden Eigenschaften auf: VZ = 164 cm³/g; MFR (230/2,16) = 29 dg/min; M_{w} = 147 500 g/mol; M_{w}/Mₙ = 2,9; breiter Schmelzbereich mit Maxima bei 142°C und 153°C. Etherextrahierbare Anteile 0,4 Gew.-%, Schüttdichte des Polymerpulves war 400 g/dm³, gute Rieselfähigkeit.

Für die Verarbeitungsversuche wurden in einer kontinuierlich betriebenen Pilotanlage in vergleichbarer Weise 960 kg des obigen Polymertyps hergestellt.

### Beispiel 10

Die Darstellung des geträgerten Metallocen-Katalysators erfolgte analog Beispiel 7, aber mit 6,54 g (10,4 mmol) rac-Dimethylsilandiylbis(2-methyl-4-phenyl-1-idenyl) zirkondichlorid und 2,24 g (4,2 mmol) rac-Dimethylsilandiylbis(2,4,6-trimethyl-1-indenyl)zirkondichlorid. Es resultierten 892 g eines frei fließenden orangefarbenen Pulvers, das laut Elementaranalyse 0,15 Gew.-% Zr und 8,0 Gew.-% Al enthielt.

### Polymerisationstest

Die Polymeriation wurde analog zu Beispiel 9 durchgeführt, es wurden jedoch 0,4 Ndm³ Wasserstoff pro dm³ Flüssigpropylen verwendet. Es resultierten 2,3 kg Polypropylen-Pulver. Die Katalysatoraktivität betrug 108 kg PP / (mmol Zrxh) oder 1,8 kg PP / (g Katxh).

Das dargestellte isotaktische Polypropylen wies die folgenden Eigenschaften auf: VZ = 136 cm³/g; MFR (230/2,16) = 39 dg/min; M_{w} = 127 500 g/mol; M_{w}/Mₙ = 2,8; breiter Schmelzbereich mit Maxima bei 143°C (Schulter) und 154°C. Etherextrahierbare Anteile 0,2 Gew.-%, Schüttdichte des Polymerpulves war 410 g/dm³, gute Rieselfähigkeit.

Für die Verarbeitungsversuche wurden in einer kontinuierlich betriebenen Pilotanlage in vergleichbarer Weise 1300 kg des obigen Polymertyps hergestellt.

### Beispiel 11

Die Darstellung des geträgerten Metallocen-Katalysators erfolgte analog Beispiel 7, aber mit 5,54 g (11,6 mmol) rac-Dimethylsilandiylbis(2-methyl-1-idenyl) zirkondichlorid und 1,44 g (3 mmol) rac-Ethylidenbis(2-ethyl-1-indenyl) zirkondichlorid. Es resultierten 884 g eines frei fließenden orangefarbenen Pulvers, das laut Elementaranalyse 0,16 Gew.-% Zr und 8,1 Gew.-% Al enthielt.

### Polymerisationstest

Die Polymeriation wurde analog zu Beispiel 7 durchgeführt. Es resultierten 2,7 kg Polypropylen-Pulver. Die Katalysatoraktivität betrug 118 kg PP / (mmol Zrxh) oder 2,1 kg PP / (g Katxh).

Das dargestellte isotaktische Polypropylen wies die folgenden Eigenschaften auf: VZ = 168 cm³/g; MFR (230/2,16) = 25 dg/min; M_{w} = 162 000 g/mol; M_{w}/Mₙ = 2,5; breiter Schmelzbereich mit Maxima bei 136°C (Schulter) und 147°C. Etherextrahierbare Anteile 0,25 Gew.-%, Schüttdichte des Polymerpulves war 420 g/dm³, gute Rieselfähigkeit.

Für die Verarbeitungsversuche wurden in einer kontinuierlich betriebenen Pilotanlage in vergleichbarer Weise 850 kg des obigen Polymertyps hergestellt.

### Verarbeitungsbeispiele

### Herstellung von Spinnvliesen (nonwoven spunbond fabrics)

### Beispiel 12

Unter Verwendung der Formmasse aus Beispiel 1 wird ein Spinnvlies hergestellt. Zum Einsatz kam dabei eine Reicofil-Anlage des Herstellers Reifenhäuser mit 1 m Vliesbahnbreite (Vorhangverfahren/Saugluftverfahren). Die Temperatur der Polymerschmelze betrug am Spinndüsenpaket 240°C, die Massetemperatur im Extruder war 250°C, die Fadengeschwindigkeit im Spinnprozeß betrug 2750 m/min, der Massedurchsatz betrug 110 kg Polymer/h.

Die auf das Transportband abgelegten Endlosfasern wurden der Thermofixierwalzeneinheit zugeführt, die Temperatur der Walzen betrug in mehreren verschiedenen Versuchseinstellungen 120 bis 160°C, wobei als Optimum für die Vliesverfestigung 144°C gefunden wurde. Die Gravurfläche (Prägefläche) der Thermofixierwalzen betrug 15 %, Rautenmuster.

Das in dieser Weise hergestellte Spinnvlies wies ein Flächengewicht von 21 g/m² auf. Die Vliesfestigkeiten betrugen in Längsrichtung 56 N/5 cm und in Querrichtung 37 N/5 cm. Die Dehnungswerte betrugen in Längsrichtung 58 % und in Querrichtung 59 %.

### Vergleichsbeispiel 1

Der Versuch wurde mit dem nicht erfindungsgemäßen Polymer 1 aus Beispiel 1 wiederholt. Als Vliesfestigkeiten wurden dabei in Längsrichtung 40 N/5 cm und in Querrichtung 28 N/5cm ermittelt, die Dehnungswerte waren Längs/Quer jeweils 60 %.

### Vergleichsbeispiel 2

Der Versuch wurde mit dem nicht erfindungsgemäßen Polymer 2 aus Beispiel 1 wiederholt. Die Temperatur der Thermofixierwalze mußte auf130°C zurückgenommen werden, um eine für dieses Polymer optimale Vliesfestigkeit zu erzielen. Als Vliesfestigkeiten wurden dabei in Längsrichtung 34 N/5 cm und in Querrichtung 20 N/5cm ermittelt, die Dehnungswerte waren in Längsrichtung 59 % und in Querrichtung 62 %.

### Vergleichsbeispiel 3

Der Versuch wurde mit dem nicht erfindungsgemäßen Hostalen PPU 1780 F1 (Fa. Hoechst AG) wiederholt. Das Polymer ließ sich mit den angewendeten hohen Fadengeschwindigkeiten nicht verarbeiten (Fadenabrisse). Wurde ein Vlies bei niedrigerer Verarbeitungsgeschwindigkeit (2000 m/min) hergestellt, so waren die Vliesfestigkeiten in Längsrichtung 33 N/5 cm und in Querrichtung 22 N/5 cm, die Dehnungswerte waren in Längsrichtung 60 % und in Querrichtung 70 %.

### Beispiele 13-15

Beispiel 12 wurde wiederholt, es wurden jedoch Spinnvliese mit Flächengewichten von 40 g/m² (Beispiel 13), 60 g/m² (Beispiel 14) und 80 g/m² (Beispiel 15) hergestellt. Ergebnisse siehe Tabelle 1

**Tabelle 1**

| Beispiel | | 13 | 14 | 15 |
|---|---|---|---|---|
| Vliesfestigkeit [N/5 cm] | längs | 120 | 182 | 263 |
| | | | | |
| | quer | 75 | 98 | 146 |
| | | | | |
| Dehnung [%] | längs | 50 | 48 | 53 |
| | | | | |
| | quer | 60 | 62 | 59 |

### Beispiel 16

Beispiel 12 wurde wiederholt, es wurde jedoch das Polymer aus Beispiel 2 verwendet. Ergebnisse siehe Tabelle 2.

### Beispiel 17

Beispiel 12 wurde wiederholt, es wurde jedoch das Polymer aus Beispiel 3 verwendet. Ergebnisse siehe Tabelle 2.

### Beispiel 18

Beispiel 12 wurde wiederholt, es wurde jedoch das Polymer aus Beispiel 4 verwendet. Ergebnisse siehe Tabelle 2.

### Beispiel 19

Beispiel 12 wurde wiederholt, es wurde jedoch das Polymer aus Beispiel 9 verwendet. Ergebnisse siehe Tabelle 2.

### Beispiel 20

Beispiel 12 wurde wiederholt, es wurde jedoch das Polymer aus Beispiel 10 verwendet. Ergebnisse siehe Tabelle 2.

### Beispiel 21

Beispiel 12 wurde wiederholt, es wurde jedoch das Polymer aus Beispiel 11 verwendet. Ergebnisse siehe Tabelle 2.

**Tabelle 2**

| Beispiel | | 16 | 17 | 18 | 19 | 20 | 21 |
|---|---|---|---|---|---|---|---|
| Vliesfestigkeit [N/5 cm] | längs | 61 | 53 | 54 | 58 | 59 | 54 |
| | | | | | | | |
| | quer | 56 | 40 | 39 | 42 | 53 | 38 |
| | | | | | | | |
| Dehnung [ % ] | längs | 51 | 59 | 58 | 48 | 50 | 57 |
| | | | | | | | |
| | quer | 54 | 59 | 59 | 57 | 51 | 58 |

### Herstellung von Faservliesen

### Beispiel 22

Verwendet wurde die Formmasse aus Beispiel 5 und der Versuch wurde auf einer Kurzspinnanlage mit rechteckigen Düsenpaketen hergestellt.

Die Temperatur der Polymerformmasse an den Spinndüsen war 225°C, die Abzugsgeschwindigkeit war 85 m/min. Die Fasern wurden auf eine Feinheit von 2,2 dtex ausgesponnen, wobei die Verstreckung 1:1,3 betrug, die Verstrecktemperatur war 130°C. Nach Kräuselung im Crimper wurde das gekräuselte Kabel bei 120°C thermisch fixiert und anschließend auf eine Länge von 5 cm geschnitten. Nach Zuführung zu einem Mischer wurden die Fasern grob aufgelöst und in einer Formierungszone (Karde) zu einem dünnen Faserflor (Vorvliesbahn) auf ein Förderband zur Thermofixierwalzeneinheit abgelegt. Die Temperaturen der Thermofixierwalzen wurden variiert, als optimale Temperatur wurde 142°C gefunden (Maximum der Vliesfestigkeit). Die Thermofixierwalzen hatten eine Gravurfläche von 22 % (Rautenmuster). Es wurde ein Vlies mit einem Flächengewicht von 20 g/m² hergestellt, die Geschwindigkeit der Vliesbahn beim Thermofixierschritt (Thermobondierschritt) betrug 140 m/min.

Die erzielbaren Vliesfestigkeiten betrugen in Längsrichtung 38 N/5 cm und in Querrichtung 17 N/5 cm. Die Dehnungswerte betrugen 37 % und 77 % in Längsrichtung bzw. Querrichtung.

### Beispiel 23

Beispiel 22 wurde mit der Polymerformmasse aus Beispiel6 wiederholt. Die Vliesfestigkeiten betrugen in Längsrichtung 39 N/5 cm und in Querrichtung 15 N/5 cm. Die Dehnungswerte betrugen 35 % bzw. 73 % (längs/quer).

### Beispiel 24

Beispiel 22 wurde mit der Polymerformmasse aus Beispiel 7 wiederholt. Die Vliesfestigkeiten betrugen in Längsrichtung 35 N/5 cm und in Querrichtung 16 N/5 cm. Die Dehnungswerte betrugen 38 % bzw. 70 % (längs/quer).

### Beispiel 25

Beispiel 22 wurde mit der Polymerformmasse aus Beispiel 8 wiederholt. Die Vliesfestigkeitswerte waren 37 N/5 cm bzw. 15 N/5 cm (längs/quer), die Dehnungswerte betrugen 37 % bzw. 71 %.

### Beispiel 26

Beispiel 22 wurde mit Polymer aus Beispiel 10 wiederholt. Die Fasern wurden auf eine Feinheit von 2,8 dtex ausgesponnen, die Verstreckung betrug 1:1,5, die Verstrecktemperatur war 135°C. Die optimale Temperatur der Thermobondierwalzen war 142°C, das so hergestellte Vlies hatte ein Flächengewicht von 22 g/m², die Geschwindigkeit der Vliesbahn beim Thermofixierschritt betrugca. 150 m/min. Die Vliesfestigkeiten betrugen in Längsrichtung 43 N/5 cm und in Querrichtung 18 N/5 cm. Die Dehnungswerte betrugen 28 % bzw. 68 %.

### Beispiel 26a

Beispiel 22 wurde mit Polymer aus Beispiel 10 wiederholt. Die Fasern wurden auf eine Feinheit von 2,8 dtex ausgesponnen, die Verstreckung betrug 1:1,5, die Verstrecktemperatur war 135°C. Die optimale Temperatur der Thermobondierwalzen war 143°C, das so hergestellte Vlies hatte ein Flächengewicht von 22 g/m², die Geschwindigkeit der Vliesbahn beim Thermofixierschritt betrug ca. 150 m/min. Die Vliesfestigkeiten betrugen in Längsrichtung 44 N/5 cm und in Querrichtung 19 N/5 cm. Die Dehnungswerte waren 30 % bzw. 70 % (längs/quer).

### Beispiele 27-29

Beispiel 26 wurde wiederholt, es wurden jedoch Vliese mit Flächengewichten von 12 g/m² (Beispiel 27), 40 g/m² (Beispiel 28) und 60 g/m² (Beispiel 29) hergestellt. Die Ergebnisse sind der Tabelle 3 zu entnehmen:

**Tabelle 3**

| Beispiel | | 27 | 28 | 29 |
|---|---|---|---|---|
| Vliesfestigkeit [N/5 cm] | längs | 25 | 76 | 128 |
| | | | | |
| | quer | 10 | 34 | 51 |
| | | | | |
| Dehnung [ % ] | längs | 35 | 30 | 28 |
| | | | | |
| | quer | 73 | 69 | 71 |

### Beispiel 30

Beispiel 26 wurde wiederholt, die Fasern wurden jedoch auf eine Feinheit von 1,4 dtex ausgesponnen. Die Temperaturen an der Thermofixierwalze wurden auf 140°C zurückgenommen. Die Vliesfestigkeiten betrugen 49 N/5 cm (längs) bzw. 26 N/5 cm (quer), die Dehnungswerte waren 28 % (längs) und 65 % (quer).

### Vergleichsbeispiele 4 bis 6

Das Beispiel 26 wurde mit den nicht erfindungsgemäßen Polymeren "Polymer 1 " aus Beispiel 1 (Vergleichsbeispiel 4), "Polymer 2" aus Beispiel 1 (Vergleichsbeispiel 5) und Hostalen PPU 1780 F1 (Fa. Hoechst AG) (Vergleichsbeispiel 6) wiederholt. Dabei wurden sowohl die in Beispiel 26 eingestellten Verarbeitungsparameter, als auch die für das jeweilige Vergleichspolymer optimierten Werte für die Verarbeitungsparameter verwendet. Mit Hostalen PPU 1780 F1 waren die maximal erzielbaren Vliesfestigkeiten 31 N/5 cm (längs) bzw. 9 N/5 cm. Die beiden mit Metallocenkatalysatoren hergestellten Polymere "Polymer 1" und "Polymer 2" erzielten 33 N/5 cm (längs) bzw. 12 N/5 cm (quer), lagen somit also deutlich unter den mit den erfindungsgemäßen Formmassen erzielbaren Vliesfestigkeiten.

### Beispiele 31-33

Als erfindungsgemäße Polymere wurden Polymer 1 und Polymer 2 aus Beispiel 1 verwendet, stabilisiert wurde die wie in Beispiel 1 zusammengesetzte Mischung (800 kg Polymer 1 und 200 kg Polymer 2) statt mit der Pentaerylthrityl-Verbindung mit 0,05 Gew.-% Irganox 1010*, 0,05 Gew.-% Irgafos 168* und 0,05 Gew.-% Calziumstearat (Beispiel 31) bzw. 0,2 Gew.-% B501 W* und 0,05 Gew.-% Calziumstearat (Beispiel 32) bzw. mit 0,05 Gew.-% Irganox* 1010 und 0,05 Gew.-% Irgafos 168* (Beispiel 33).
Die Granulierung der Mischungen erfolgte wie in Beispiel 1 beschrieben. Verarbeitet wurden die Formmassen zu Spinnvliesen wie in Beispiel 12 beschrieben. Die so hergestellten Vliese wiesen in Längsrichtung Festigkeiten von 54-57 N/5 cm und in Querrichtung 36-40 N/5 cm auf. Die Dehnungswerte lagen bei 54 bis 60 % (Längs- und Querrichtung).

| | |
|---|---|
| Irganos 1010* | Handelsname der Ciba Geigy AG; Pentaerythrityl-tetrakis[3-(3,S-di-tert.butyl-4-hydrophenyl)-propionat] |
| Irgafos168* | Handelsname der Ciba-Geigy AG; Tris(2,4-di-tert.butylphenyl)phosphonit |
| B 501 W* | Handelsname der Ciba Geigy AG; Mischung aus: 50 Gew.-% Irgafos 168, 25 % PE-Wachs und 25 Gew.-% Calzium-3,5-di-tert.butyl-4-hydroxibenzyl-monoethyl-phosphonat |

## Patentansprüche

1. Verfahren zur Herstellung von Vliesen aus mindestens einer Polyolefinformmasse, wobei die Polyolefinformmasse mindestens zwei unterschiedliche Polyolefine enthält, die durch Polymerisation oder Copolymerisation von Olefinen der Formel R⁹CH = CHR⁶, worin R⁹ und R⁶ gleich oder verschieden sind und ein Wasserstoffatom oder einen Alkylrest mit 1 bis 14 C-Atomen bedeuten oder R⁹ und R⁶ mit den sie verbindenden Atomen ein Ringsystem bilden, hergestellt werden, und wobei die Polyolefinformmasse einen etherextrahierbaren Anteil kleiner als 2 Ges.-% aufweist und deren Polydispersität (M_{w}/Mₙ) von 1,8 bis 3,0 beträgt.

2. Verfahren zur Herstellung von Vliesen nach Anspruch 1, wobei die Polyolefinformmasse mindestens ein Polyolefin mit hoher Isotaktizität und von 5 bis 60 Gew.-%, mindestens eines weiteren Polyolefins mit niedriger Isotaktizität enthält.

3. Verfahren zur Herstellung von Vliesen nach Anspruch 1 oder 2, wobei die Polyolefinformmasse einen MFI (23012,16) von 5 bis 1000 dg/min, eine Molmasse (M_{w}) von 75 000 bis 350 000 g/mol und eine Viskositätszahl von 70 bis 250 cm³/g aufweist.

4. Verfahren zur Herstellung von Vliesen nach einem oder mehreren der Ansprüche 1 bis 3, wobei die Schmelzpunkte der einzelnen Polyolefine sich um mindestens 5°C unterscheiden, eines der Polyolefine eine isotaktische Blocklänge von mindestens 40 Monomereinheiten und das andere Polyolefin eine isotaktische Blocklänge von 10 bis 80 Monomereinheiten aufweist, wobei der Unterschied der Blocklängen der Polyolefine in der Formmasse mindestens 5 beträgt.

5. Verfahren zur Herstellung von Vliesen nach einem oder mehreren der Ansprüche 1 bis 3, wobei mindestens ein Polyolefin eine isotaktische Blocklänge von 50 bis 200 aufweist und das andere Polyolefin ein Copolymer mit einem Comanomergehaft von 0,5 bis 60 Gew.-% ist.

6. Verfahren zur Herstellung von Vliesen nach einem oder mehreren der Ansprüche 1 bis 3, wobei mindestens ein Polyolefine isotaktisch aufgebaut ist und eine isotaktische Blocklänge von 40 bis 200 aufweist und das andere Polyolefin syndiotaktisch aufgebaut ist und sich die Schmelzpunkte des syndiotaktisch aufgebauten und des isotaktisch aufgebauten Polyolefins um mindestens 10°C unterscheiden.

7. Verfahren zur Herstellung von Vliesen nach einem oder mehreren der Ansprüche 1 bis 6, wobei in der Polyolefinformmasse Zusätze wie Nukleierungsmittel, Talkum, Natriumbenzoat, Stearate oder Sorbitolderivate, Stabilisatoren, Antioxidantien, UV-Absorber, Lichtschutzmittel, Metalldesaktivatoren, Radikalfänger, Gleitmittel, Emulgatoren, Pigmente, optische Aufheller, Flammschutzmittel oder Antistatika enthalten sind.

8. Polyolefinformmasse, enthaltend mindestens zwei unterschiedlichen Polyolefine, die durch Polymerisation oder Copolymerisation von Olefinen der Formel R⁹CH = CHR⁶, worin R⁹ und R⁶ gleich oder verschieden sind und ein Wasserstoffatom oder einen Alkylrest mit 1 bis 14 C-Atomen bedeuten oder R⁹ und R⁶ mit den sie verbindenden Atomen ein Ringsystem bilden, hergestellt werden, wobei die Polyolefinformmasse einen etherextrahierbaren Anteil kleiner als 2 Gew.-% aufweist und deren Polydispersität (M_{w}/Mₙ) von 1,8 bis 3,0 beträgt und wobei mindestens ein Polyolefin isotaktisch aufgebaut ist und eine isotaktische Blocklänge von 40 bis 200 aufweist und das andere Polyolefin syndiotaktisch aufgebaut ist und sich die Schmelzpunkte des syndiotaktisch aufgebauten und des isotaktisch aufgebauten Polyolefins um mindestens 10°C unterscheiden.

9. Vlies erhältlich durch ein Verfahren nach einem oder mehreren der Ansprüchen 1 bis 7.

10. Verwendung eines Vlieses nach Anspruch 9 als Hygienevlies, Agrarvlies Filtervlies, Bauvlies oder im Textilbereich.

## Claims

1. A process for producing nonwovens from at least one polyolefin molding composition, wherein the polyolefin molding composition comprises at least two different polyolefins which are produced by polymerization or copolymerization of olefins of the formula R⁹CH = CHR⁶, where R⁹ and R⁶ are the same or different and represent a hydrogen atom or an alkyl radical of 1 to 14 carbon atoms or R⁹ and R⁶ combine with their linking atoms to form a ring system, and wherein the polyolefin molding composition has an ether-extractable fraction of less than 2% by weight and its polydispersity (M_{w}/Mₙ) is in the range from 1.8 to 3.0.

2. A process for producing nonwovens according to claim 1, wherein the polyolefin molding composition comprises at least one polyolefin of high isotacticity and from 5 to 60% by weight of at least one further polyolefin of low isotacticity.

3. A process for producing nonwovens according to claim 1 or 2, wherein the polyolefin molding composition has an MFI (230/2.16) from 5 to 1000 dg/min, a molar mass (M_{w}) from 75 000 to 350 000 g/mol and a viscosity number from 70 to 250 cm³/g.

4. A process for producing nonwovens according to one or more of claims 1 to 3, wherein the melting points of the individual polyolefins differ by at least 5°C, one of the polyolefins has an isotactic block length of at least 40 monomer units and the other polyolefin has an isotactic block length of 10 to 80 monomer units, the difference in the block lengths of the polyolefins in the molding composition being at least 5.

5. A process for producing nonwovens according to one or more of claims 1 to 3, wherein at least one polyolefin has an isotactic block length of 50 to 200 and the other polyolefin is a copolymer having a comonomer content of 0.5 to 60% by weight.

6. A process for producing nonwovens according to one or more of claims 1 to 3, wherein at least one polyolefin is isotactic and has an isotactic block length of 40 to 200 and the other polyolefin is syndiotactic and the melting points of the syndiotactic and isotactic polyolefins differ by at least 10°C.

7. A process for producing nonwovens according to one or more of claims 1 to 6, wherein the polyolefin molding composition comprises additives such as nucleants, talc, sodium benzoate, stearates or sorbitol derivatives, stabilizers, antioxidants, UV absorbers, light protectants, metal deactivators, radical scavengers, lubricants, emulsifiers, pigments, optical brighteners, flame retardants or antistats.

8. A polyolefin molding composition comprising at least two different polyolefins which are produced by polymerization or copolymerization of olefins of the formula R⁹CH = CHR⁶, where R⁹ and R⁶ are the same or different and represent a hydrogen atom or an alkyl radical of 1 to 14 carbon atoms or R⁹ and R⁶ combine with their linking atoms to form a ring system, and wherein the polyolefin molding composition has an ether-extractable fraction of less than 2% by weight and its polydispersity (M_{w}/Mₙ) is in the range from 1.8 to 3.0 and wherein at least one polyolefin is isotactic and has an isotactic block length of 40 to 200 and the other polyolefin is syndiotactic and the melting points of the syndiotactic and isotactic polyolefins differ by at least 10°C.

9. A nonwoven obtainable by a process according to one or more of claims 1 to 7.

10. The use of a nonwoven according to claim 9 as a hygiene fabric, as an agrifabric, as a filter fabric, as a building construction fabric or in the textile sector.

## Revendications

1. Procédé pour la fabrication de non-tissés comprenant au moins une matière moulable à base de polyoléfines, la matière moulable à base de polyoléfines comprenant au moins deux polyoléfines différentes, fabriquées par polymérisation ou copolymérisation d'oléfines de formule R⁹CH = CHR⁶, dans laquelle R⁹ et R⁶ sont identiques ou différents et représentent un atome, d'hydrogène ou un radical alkyle avec 1 à 14 atomes C, ou dans laquelle R⁹ et R⁶ forment une combinaison cyclique avec les atomes qui les lient, et la matière moulable à base de polyoléfines comportant une part pouvant être extraite à l'éther inférieure à 2 % en poids, et dont la polydispersité (M_{w}/Mₙ) va de 1,8 à 3,0.

2. Procédé pour la fabrication de non-tissés selon la revendication 1, la matière moulable à base de polyoléfines comprenant au moins une polyoléfine présentant une isotacticité élevée et au moins de 5 à 60 % en poids d'une autre polyoléfine présentant une isotacticité inférieure.

3. Procédé pour la fabrication de non-tissés selon la revendication 1 ou 2, la matière moulable à base de polyoléfines comportant un indice de fluidité (230/2,16) allant de 5 à 1000 dg/min, une masse molaire (M_{w}) allant de 75 000 à 350 000 g/mol et un indice de viscosité allant de 70 à 250 cm³/g.

4. Procédé pour la fabrication de non-tissés selon l'une ou plusieurs des revendications 1 à 3, les points de fusion de chacune des polyoléfines se différenciant d'au moins 5°C, une des polyoléfines comportant une longueur de bloc isotactique d'au moins 40 motifs monomères, et l'autre polyoléfine comportant une longueur de bloc isotactique allant de 10 à 80 motifs monomères, la différence des longueurs de bloc des polyoléfines dans la matière moulable étant d'au moins 5.

5. Procédé pour la fabrication de non-tissés selon l'une ou plusieurs des revendications 1 à 3, au moins une polyoléfine comportant une longueur de bloc isotactique allant de 50 à 200, et l'autre polyoléfine étant un copolymère présentant une teneur en comonomères allant de 0,5 à 60 % en poids.

6. Procédé pour la fabrication de non-tissés selon l'une ou plusieurs des revendications 1 à 3, au moins une polyoléfine étant constituée de manière isotactique et comportant une longueur de bloc isotactique allant de 40 à 200, et l'autre polyoléfine étant constituée de manière syndiotactique, et les points de fusion des polyoléfines constituées de manière syndiotactique et isotactique se différenciant d'au moins 10°C.

7. Procédé pour la fabrication de non-tissés selon l'une ou plusieurs des revendications 1 à 6, la matière moulable à base de polyoléfines comprenant des ingrédients tels que des moyens de nucléarisation, du talc, du benzoate de sodium, des stéarates ou des dérivés de sorbitol, des stabilisateurs, des anti-oxydants, des absorbants d'UV, des agents de photoprotection, des désactivateurs métalliques, des capteurs de radicaux, des agents de démoulage, des agents émulsifiants, des pigments, des sensibilisateurs optiques, des agents ignifuges ou des antistatiques.

8. Poudre de moulage à base de polyoléfines, comprenant au moins deux polyoléfines différentes fabriquées par la polymérisation ou la copolymérisation d'oléfines de formule R⁹CH = CHR⁶, dans laquelle R⁹ et R⁶ sont identiques ou différents et représentent un atome d'hydrogène ou un radical alkyle avec 1 à 14 atomes C, ou dans laquelle R⁹ et R⁶ forment une combinaison cyclique avec les atomes qui les lient, la matière moulable à base de polyoléfines comportant une part pouvant être extraite à l'éther inférieure à 2 % en poids, et dont la polydispersité (M_{w}/Mₙ) va de 1,8 à 3,0, et au moins une polyoléfine étant constituée de manière isotactique et comportant une longueur de bloc isotactique de 40 à 200 et l'autre polyoléfine étant constituée de manière syndiotactique et les points de fusion des polyoléfines constituées de manière syndiotactique et isotactique se différenciant d'au moins 10°C.

9. Non-tissé pouvant être obtenu par un procédé selon l'une ou plusieurs des revendications 1 à 7.

10. Utilisation d'un non-tissé selon la revendication 9 en tant que non-tissé hygiénique, non-tissé agraire, non-tissé filtrant, non-tissé structural ou dans le secteur textile.
